# EUROPEAN PATENT SPECIFICATION

(11) **EP 2 854 796 B1**
(45) Date of publication and mention of the grant of the patent: **14.11.2018**
(21) Application number: 13797528.0
(22) Date of filing: 03.06.2013
(51) Int. Cl.: A61K 31/343, A61K 31/506, A61K 31/661, A61K 31/675, A61K 31/69, A61K 45/06, A61P 35/00

(54) **COMBINATIONS OF A VASCULAR DISRUPTING AGENT AND A HYPOXIA-TARGETING AGENT FOR USE IN THE TREATMENT OF CANCER**
KOMBINATIONEN AUS EINEM VASKULÄRDISRUPTIONSMITTEL UND EINEM HYPOXIE ABZIELENDEN MITTEL ZUR BEHANDLUNG VON KREBS
COMBINAISONS CONTENANT UN AGENT DE PERTURBATION VASCULAIRE ET UN AGENT CIBLANT L'HYPOXIE DESTINÉES AU TRAITEMENT DU CANCER

(30) Priority: 01.06.2012 AU 2012902291
(43) Date of publication of application: 08.04.2015
(73) Proprietor: Bionomics Limited, Thebarton, South Australia 5031 (AU)
(72) Inventor: INGLIS, Daniel, J., Thebarton, South Australia 5031 (AU); LAVRANOS, Tina, C., Thebarton, South Australia 5031 (AU); KREMMIDIOTIS , Gabriel, Thebarton, South Australia 5031 (AU)
(74) Representative: J A Kemp
(86) International application number: PCT/AU2013/000581
(87) International publication number: WO 2013/177633

(56) References cited:
- EP-B1- 1 984 333
- WO-A2-2012/009288
- CA-A1- 2 686 587
- US-A1- 2011 130 367
- US-A1- 2012 087 913
- INGLIS, DANIEL J; LAVRANOS, TINA C; BEAUMONT, DONNA M; LESKE, ANNABELL F; BROWN, CHLOE K; HALL, ALLISON J; KREMMIDIOTIS, GABRIEL: "The vascular disrupting agent BNC105 potentiates the efficacy of VEGF and mTOR inhibitors in renal and breast cancer", CANCER BIOLOGY AND THERAPY, vol. 15, no. 11, 2014, pages 1552-1560, XP009185993,
- MONAGHAN, K. ET AL.: 'CYT997 causes apoptosis in human multiple myeloma.' INVEST NEW DRUGS. vol. 29, no. 2, April 2011, pages 232 - 8, XP019881448
- MASUNAGA, S. ET AL.: 'Dependency of the effect of a vascular disrupting agent on sensitivity to tirapazamine and gamma-ray irradiation upon the timing of its administration and tumor size, with reference to the effect on intratumor quiescent cells.' J CANCER RES CLIN ONCOL. vol. 133, no. 1, January 2007, pages 47 - 55, XP019460827
- MASUNAGA, S ET AL.: 'Combination of the antivascular agent ZD6126 with hypoxic cytotoxin treatment, with reference to the effect on quiescent tumor cells and the dependency on p53 status of tumor cells.' ONCOL REP. 1791-2431 vol. 14, no. 2, August 2005, pages 393 - 400, XP008175504
- KENDREW, J. ET AL.: 'Anti-tumour and anti-vascular effects of cediranib (AZD2171) alone and in combination with other anti-tumour therapies.' CANCER CHEMOTHER PHARMACOL. vol. 71, no. 4, April 2013, pages 1021 - 32, XP035339852

## Description

The present invention relates generally to new chemical combinations for use in the treatment of cancers.

### BACKGROUND OF THE INVENTION

Cancer is typically treated with either chemotherapy and/or radiation therapy. While often effective to destroy a significant amount of tumour cells, such therapies often leave behind a number of tumour cells that are resistant to the treatment. These resistant cells can proliferate to form new tumors that are then resistant to treatment. The use of known combinations of chemotherapeutic drugs has given rise to multidrug resistant ('MDR') tumour cells.

The mode of proliferative diseases, such as cancer, is multi-factorial. For instance, research over the last forty years has led to the realisation that cytotoxic agents (or anti-proliferative agents) includes anti-metabolic agents which interfere with microtubule formulation, alkylating agents which are able to cross-link DNA, platinum based agents which are able to interfere with DNA alkylation by blocking DNA replication, antitumor antibiotic agents, topoisomerase inhibitors, etc. In the treatment of such diseases drugs with different mechanisms may be combined (i.e, combination therapies) with beneficial effects including the effective treatment of MDR tumour cells and the minimisation of side effects such as undesirable cytotoxicity. The difficulty here is though that not all known antiproliferative agents provide useful or beneficial effects in combination and accordingly research in many laboratories is presently focused on developing new and useful anti-proliferative combination partners.

The following four documents were cited in the International Search Report:
US 2012/087913, describing the induction of tumor hypoxia for cancer therapy.
WO 2012/009288, describing the administration of hypoxia activated prodrugs and antiangiogenic agents for the treatment of cancer.
CA 2686587, describing combination therapy for treating proliferative diseases.
Invest. New Drugs 2011, 29 (2), 232, describing that CYT 997 causes apotosis in human multiple myeloma.

### SUMMARY OF THE INVENTION

The present inventors have found that the use of a vascular disrupting agent (VDA), in particular BNC105, in conjunction with an agent which targets hypoxia, in particular Pazopanib or Bortezomib, provides a highly effective combination in the treatment of certain proliferative diseases. Indeed a synergistic effect has been shown with the combinations of BNC105 and Pazopanib and BNC105 and Bortezomib.

Specificaly, the present invention provides a pharmaceutical combination for use in treating a proliferative disease selected from renal cancer, lung cancer, breast cancer, prostate cancer, and pancreatic cancer, comprising:
a) a vascular disrupting agent (VDA) which is a compound having the structure 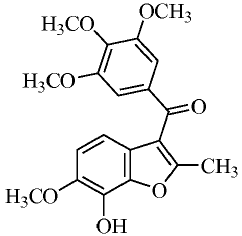 or a salt or solvate thereof, or 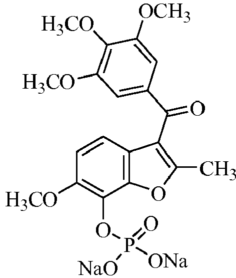 or a salt or solvate thereof; and
(b) at least one hypoxia targeting agent selected from Pazopanib, Bortezomib or TH-302.

The present invention further provides a kit of parts comprising a pharmaceutical combination for use according to the previous paragraph.

The present disclosure also provides a method for treating a proliferative disease selected from renal cancer, lung cancer, breast cancer, prostate cancer, and pancreatic cancer, including the step of administering to a patient in need thereof: (a) a VDA, and (b) at least one other agent which targets hypoxia, wherein (a) and (b) are as defined above.

It is believed that the effects in treating the above-mentioned proliferative diseases with a combination which comprises: (a) a VDA, and (b) at least one other agent which targets hypoxia, wherein (a) and (b) are as defined above, are greater than the effects that can be achieved with either (a) or (b) alone. That is, the present combinations are believed to possess an additive or synergistic effect.

### BRIEF DESCRIPTION OF THE FIGURES

**Figure 1** Rationale for therapy involving BNC105 in combination with tumor hypoxia targeting agents.
**Figure 2** Survival of mice bearing RENCA kidney tumours treated with BNC105P, Pazopanib or BNC105P + Pazopanib.
**Figure 3** Right kidney weight (mg) ± SEM of mice bearing RENCA kidney tumours treated with BNC105P, Pazopanib or BNC105P + Pazopanib.

### DETAILED DESCRIPTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

### Combination partner (a); vascular disrupting agent (VDA)

As used herein the term "vascular disrupting agents" refers to compounds which are able to disrupt vasculature, in particular tumour vasculature..

Examples of VDA's include:

### Synthetic compounds

ABT-751 (E7010, Abbott)
MPC-6827 (Azixa™, Myriad Pharmaceuticals)
CYT997 (Cytopia)
MN-029 (Denibulin, MediciNova/Angiogene)
EPC2407 (EpiCept)

### Natural products derivatives

Combretastatins
CA4 (Zybrestat™, OXiGENE)
Oxi4503 (OXiGENE)
AVE8062 (AC7700, Sanofi Aventis)
NPI-2358 (Nereus Pharmaceuticals)
TZT1027 (Soblidotin)

VDAs are important in the treatment of cancers primarily as a result of their capacity to selectively shut down blood flow through a tumour.

VDAs interfere with microtubule integrity, leading to cytoskeletal changes of the endothelial cells that line the blood vessels of the tumour. As a result, these usually flat cells become more rounded, and lose their cell to cell contact. These events lead to narrowing of tumour blood vessels and ultimately occlusion of blood flow through the vessels. The tumour selectivity associated with these agents results from the fact that tumour vasculature is weaker and more prone to collapse than normal vasculature. Nonetheless, a number of the dose limiting toxicities associated with VDAs are due to a reduction in blood flow in healthy tissues.

VDAs may act at the colchicine binding site and be based on annulated furans (e.g., benzofurans, furo[2,3-d]pyrimidin-2(1H)-ones, etc), benzothiophene and indole structural scaffolds, such as those disclosed in US 7,456,214, US 7,429,681, US 7,071,190, US 6,849,656, US 5,886,025, US 6,162,930, US 6,350,777, US 5,340,062, WO 06/084338, WO 02/060872, WO 07/087684, and WO 08/070908.

VDAs are also disclosed in WO 06/084338, WO 07/087684, or WO 08/070908.

VDAs may be prepared by known methods including those disclosed in WO 02/060872 and WO 07/087684.

The VDA for use in the present invention is a compound of formula (III) or a salt or solvate thereof 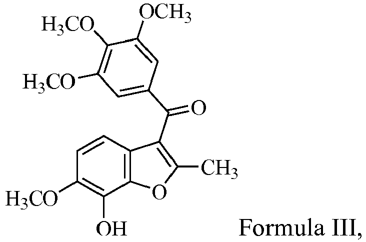 or a compound of the following formula 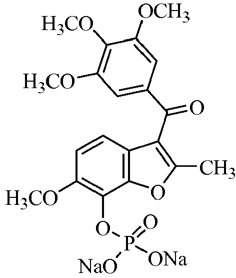 or a salt or solvate thereof.

The compound of formula (III) (2-Methyl-7-hydroxy-3-(3,4,5-trimethoxybenzoyl)-6-methoxybenzofuran) can be prepared by the synthetic methodology described in PCT/AU2007/000101 (WO 07/087684).

The VDAs for use in the invention such as the compound of formula III have been observed to be potent tubulin polymerisation inhibitors (TPIs). An important aspect of the VDAs is the combination of the specific C-6 and C-7 substituents together with the C-2 Q-group (methyl) which appears to confer greater potency and selectivity when compared to other structurally related TPI compounds. In these compounds selectivity is not simply reliant on the predisposition of tumour vasculature towards collapse when challenged with the VDA but on a capacity of the VDA to distinguish between tumour endothelial cells and normal endothelial cells. Normal endothelial cells, found in healthy tissues, are in a "quiescent" state and tumour endothelial cells are in an "activated" state. Most VDAs do not distinguish between these two states, for example, Combretastatin A4 (CA4) is equally potent against quiescent and activated endothelial cells. However, the VDAs for use in the invention and particularly the compound of formula III show selectivity towards tumor endothelial cells (activated) over normal endothelial cells (quiescent).

It will be appreciated that the VDAs of the invention such as the compound of formula III can be administered to a subject as a pharmaceutically acceptable salt thereof. Suitable pharmaceutically acceptable salts include salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, maleic, citric, lactic, mucic, gluconic, benzoic, succinic, oxalic, phenylacetic, methanesulphonic, toluenesulphonic, benezenesulphonic, salicyclic sulphanilic, aspartic, glutamic, edetic, stearic, palmitic, oleic, lauric, pantothenic, tannic, ascorbic and valeric acids.

Base salts include those formed with pharmaceutically acceptable cations, such as sodium, potassium, lithium, calcium, magnesium, ammonium and alkylammonium. In particular, the present invention includes cationic salts eg sodium or potassium salts, or alkyl esters (eg methyl, ethyl) of the phosphate group.

The VDA for use in the invention, such as the compound of formula III (or a salt or C-7 disodium phosphate ester prodrug thereof), may be in crystalline form either as the free compound or as a solvate (e.g. hydrate). Methods of solvation are generally known within the art.

### Combination partner (b); hypoxia targeting agent

The induction of hypoxia in a tumour results in upregulation of a number of molecules including CXCL12, MMP-9, Hifla, VEGFA, p-mTOR, PERK, p-eIF2α, GLUT1 and other hypoxia activated agents.

Accordingly, agents that inhibit or target one or more molecules upregulated by tumour hypoxia are encompassed by the meaning of "hypoxia targeting agent" as used herein.

Examples of hypoxia targeting agents are Retaspimycin HCl, Geldanamycin derivatives, Sunitib, Pazopanib, Ridaforolimus, Bortezomib, 2-Deoxyglucose, Lonidamine, Imatinib, TH-302 with or without Chk1 inhibitors, PR-104, CXCR4-SDF-1 targeting agents, BMS-936564 and Tirapazamine. The pharmaceutical combination for use in the invention comprises at least one hypoxia targeting agent selected from Pazopanib, Bortezomib or TH-302.

The suitability of any hypoxia targeting combination partner will often depend on the mode of delivery, however, particularly preferred agents are Pazopanib and Bortezomib. The preferred combinations of agents are BNC105 and Pazopanib and BNC105 and Bortezomib. Typically the BNC105 will be used in the form of a phosphate salt.

### Proliferative Diseases

As used herein the term "proliferative disease" broadly encompasses any neoplastic disease including those which are potentially malignant (pre-cancerous) or malignant (cancerous). The term therefore encompasses the treatment of tumours.

Accordingly, the term "tumour" is used generally to define any malignant cancerous or pre-cancerous cell growth, and may include leukaemias and carcinomas such as melanomas, colon, lung, ovarian, skin, breast, pancreas, pharynx, brain prostate, CNS, and renal cancers, as well as other cancers.

In a preferred embodiment the invention provides a combination of (a) and (b) for use in the treatment of breast adenocarcinoma, lung carcinoma, pancreatic adenocarcinoma, prostate carcinoma, and renal cell adenocarcinoma.

In a preferred aspect the invention provides a combination of (a) and (b) for use in the treatment of renal cancer, and in particular metastatic renal cell carcinoma.

### The combination of (a) and (b)

Without wishing to be bound to any particular theory it is postulated that VDA's induce hypoxia in highly vascularised tumors which can drive angiogenic/survival responses. Combination partners (b) target various hypoxia response pathways. Therefore inhibition using combination partner (b) directed against tumor hypoxia may yield a beneficial additive or synergistic effect through limiting the induction of the angiogenic/survival responses when combined with combination partner (a).

The present disclosure therefore provides a method of treating a proliferative disease as defined above, which comprises treating tumours and which comprises the administration of an effective amount of (a) a VDA in combination with an effective amount of (b) at least one hypoxia targeting agent, where (a) and (b) are as defined above.

An "effective amount" is intended to mean that the amount of each combination partner, when administered to a mammal (in particular a human) in need of such treatment, is sufficient to effect treatment for the particular proliferative disease. Thus, for example, a therapeutically effective amount of the compound of combination partner (a) (or the pharmaceutically acceptable salt or solvate thereof) is a quantity sufficient to synergise or potentiate the activity of the hypoxia targeting agent (or *vice versa*) such that a targeted disease is reduced or alleviated.

This may include at least partially attaining the desired effect, or delaying the onset of, or inhibiting the progression of, or halting or reversing altogether the onset or progression of the particular tumour being treated.

Clinical studies such as open-label, dose escalation studies in patients with proliferative diseases may include studies to prove the synergism of the active ingredients of the combination. The beneficial and/or synergistic effects can be determined directly through the results of these studies which are known as such to a person skilled in the art. These studies are also able to compare the effects of a monotherapy using the active ingredients and a combination for use in the invention. Preferably, the dose of combination partner (a) may be escalated until the Maximum Tolerated Dosage (MTD) is reached, and agent (b) is administered as a fixed dose. Alternatively, combination partner (a) is administered in a fixed dose and the dose of agent (b) is escalated. Each patient may receive doses of agent (a) either daily or intermittent. The efficacy of the treatment can be determined in such studies, e.g., after 6, 12, 18 or 24 weeks by evaluation of symptom scores every 9 weeks.

The administration of the pharmaceutical combination for use in the present invention may result not only in a beneficial effect, e.g., an additive or synergistic therapeutic effect, for instance, with regard to alleviating, delaying progression of or inhibiting the symptoms, but also in further surprising beneficial effects. Such other effects may include fewer side effects, an improved quality of life or a decreased morbidity, compared with a monotherapy applying only one of the pharmaceutically active ingredients used in the combination for use in the present invention.

A further benefit of the invention is that lower doses of the active ingredients of the combination can be used. The dosages need not only be smaller but may also be applied less frequently, which may diminish the incidence or severity of side effects.

The term "administration" relates to the co-administration of the combination partners to a single patient, and are intended to include treatment regimens in which the agents are not necessarily administered by the same route of administration or at the same time. Accordingly, combination partners (a) and (b) may be administered together, one after the other or separately in one combined unit dosage form or in two separate unit dosage forms. The unit dosage form may also be a fixed combination such as a pharmaceutical composition which comprises both partner (a) (or the salt, solvate or prodrug thereof) and partner (b).

In particular, a therapeutically effective amount of each of the combination partner of the combination for use in the invention may be administered simultaneously or sequentially and in any order, and the components may be administered separately or as a fixed combination.

For example, the method of preventing or treating proliferative diseases according to the present disclosure may comprise: (i) administration of partner (a) in free or pharmaceutically acceptable salt form; and (ii) administration of partner (b) in free or pharmaceutically acceptable salt form, simultaneously or sequentially in any order, in jointly therapeutically effective amounts, preferably in synergistically effective amounts, e.g., in daily or intermittent dosages corresponding to the amounts described herein. The individual combination partners of the combination for use in the invention may be administered separately at different times during the course of therapy or concurrently in divided or single combination forms. Furthermore, the term administering also encompasses the use of a pro-drug of a combination partner that converts in vivo to the combination partner as such. The present disclosure is therefore to be understood as embracing all such regimens of simultaneous or alternating treatment and the term "administering" is to be interpreted accordingly.

As such it will be appreciated that the combination partners may be presented as a "kit of parts" for use in the treatment of a proliferative disease as defined above (e.g., tumour therapy). The kit may comprise a package where the combination partners are supplied separately for co-administration with instructions for use in the particular therapy.

The effective dosage of each of the combination partners employed in the combination for use in the invention may vary depending on the particular compound or pharmaceutical composition employed, the mode of administration, the condition being treated, the severity of the condition being treated. Thus, the dosage regimen of the combination for use in the invention is selected in accordance with a variety of factors including the route of administration and the renal and hepatic function of the patient. A physician of ordinary skill can readily determine and prescribe the effective amount of the single active ingredients required to alleviate, counter or arrest the progress of the condition.

Daily dosages for combination partners (a) and (b) will, of course, vary depending on a variety of factors, e.g., the compound chosen, the particular condition to be treated and the desired effect. In general, however, satisfactory results are achieved on administration of agent (a) at daily dosage rates of 0.05 to 20 mg/kg per day, particularly 1 to 20 mg/kg per day, e.g. 0.4 to 16 mg/kg per day, as a single dose or in divided doses. Combination partner (a) and partner (b) may be administered by any conventional route, in particular enterally, e.g., orally, e.g., in the form of tablets, capsules, drink solutions or parenterally, e.g., in the form of injectable solutions or suspensions. Suitable unit dosage forms for oral administration comprise from 0.02 to 50 mg active ingredient, usually 0.1 to 30 mg and 2 to 25 mg, 4 to 20 mg e.g. combination partner (a) or (b), together with one or more pharmaceutically acceptable diluents or carriers therefore.

Combination partner (b) may be administered to a human in a daily dosage range of 0.5 to 1000 mg. Suitable unit dosage forms for oral administration comprise from 0.1 to 500 mg active ingredient, preferably 5-50 mg/day, more preferably 5-20 mg/day, and most preferably 7-12 mg/day, together with one or more pharmaceutically acceptable diluents or carriers therefore. Methods and administration regimes for delivery known hypoxia targeting agents would be known to the skilled clinician.

For instance, an administration regime may include adding the TPI (e.g., compound of formula III) at an assigned dose level by I.V. on days 1 and 8 (of a 21 day cycle) where the hypoxia targeting agent is given as an oral daily dose (e.g., 10 mg/day). In this embodiment the compound of formula (III) may be dosed at a level of between 4 to 16 mg/m².

The administration of a pharmaceutical combination for use in the invention results not only in a beneficial effect, e.g., an additive or synergistic therapeutic effect, e.g., with regard to inhibiting the growth of tumors, but also in further surprising beneficial effects, e.g., less side effects, an improved quality of life or a decreased morbidity, compared to a monotherapy applying only one of the pharmaceutically active ingredients used in the combination for use in the invention.

A further benefit is that lower doses of the active ingredients of the combination for use in the invention can be used, e.g., that the dosages need not only often be smaller but are also applied less frequently, or can be used in order to diminish the incidence of side effects. This is in accordance with the desires and requirements of the patients to be treated.

Combinations of partners (a) and (b) may be combined, independently or together, with one or more pharmaceutically acceptable carriers and, optionally, one or more other conventional pharmaceutical adjuvants and administered enterally, e.g., orally, in the form of tablets, capsules, caplets or parenterally, e.g., intraperitoneally or intravenously, in the form of sterile injectable solutions or suspensions. The enteral and parenteral compositions may be prepared by conventional means.

The pharmaceutical compositions for separate administration of combination partner (a) and partner (b) or for the administration in a fixed combination (i.e., a composition), according to the invention may be prepared in a manner known in the art and are those suitable for enteral, such as oral or rectal, and parenteral administration to mammals (warm-blooded animals), particularly humans, comprising a therapeutically effective amount of at least one pharmacologically active combination partner alone, e.g., as indicated above, or in combination with one or more pharmaceutically acceptable carriers or diluents, especially suitable for enteral or parenteral application.

Suitable pharmaceutical compositions contain, e.g., from 0.1% to 99.9%, preferably from 1 % to 60%, of the active ingredient(s).

The composition may contain any suitable carriers, diluents or excipients. These include all conventional solvents, dispersion media, fillers, solid carriers, coatings, antifungal and antibacterial agents, dermal penetration agents, surfactants, isotonic and absorption agents. It will be understood that the compositions for use in the invention may also include other supplementary physiologically active agents.

The carrier must be pharmaceutically "acceptable" in the sense of being compatible with the other ingredients of the composition and not injurious to the subject. Compositions include those suitable for oral, rectal, nasal, topical (including buccal and sublingual), vaginal or parental (including subcutaneous, intramuscular, intravenous and intradermal) administration. The compositions may conveniently be presented in unit dosage form and may be prepared by any methods well known in the art of pharmacy. Such methods include the step of bringing into association the active ingredient with the carrier which constitutes one or more accessory ingredients. In general, the compositions are prepared by uniformly and intimately bringing into association the active ingredient with liquid carriers or finely divided solid carriers or both, and then if necessary shaping the product.

Compositions for use in the present invention suitable for oral administration may be presented as discrete units such as capsules, sachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g inert diluent, preservative disintegrant (e.g. sodium starch glycolate, cross-linked polyvinyl pyrrolidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach.

Compositions suitable for topical administration in the mouth include lozenges comprising the active ingredient in a flavoured base, usually sucrose and acacia or tragacanth gum; pastilles comprising the active ingredient in an inert basis such as gelatine and glycerin, or sucrose and acacia gum; and mouthwashes comprising the active ingredient in a suitable liquid carrier.

Compositions suitable for topical administration to the skin may comprise the compounds dissolved or suspended in any suitable carrier or base and may be in the form of lotions, gel, creams, pastes, ointments. Suitable carriers include mineral oil, propylene glycol, polyoxyethylene, polyoxypropylene, emulsifying wax, sorbitan monostearate, polysorbate 60, cetyl esters wax, cetearyl alcohol, 2-octyldodecanol, benzyl alcohol and water. Transdermal patches may also be used to administer the compounds of the invention.

Compositions for rectal administration may be presented as a suppository with a suitable base comprising, for example, cocoa butter, glycerin, gelatine or polyethylene glycol.

Compositions suitable for vaginal administration may be presented as pessaries, tampons, creams, gels, pastes, foams or spray formulations containing in addition to the active ingredient such carriers as are known in the art to be appropriate.

Compositions suitable for parenteral administration include aqueous and non-aqueous isotonic sterile injection solutions which may contain anti-oxidants, buffers, bactericides and solutes which render the composition isotonic with the blood of the intended recipient; and aqueous and non-aqueous sterile suspensions which may include suspending agents and thickening agents. The compositions may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage compositions are those containing a daily dose or unit, daily sub-dose, as herein above described, or an appropriate fraction thereof, of the active ingredient.

It should be understood that in addition to the active ingredients particularly mentioned above, the compositions for use in this invention may include other agents conventional in the art having regard to the type of composition in question, for example, those suitable for oral administration may include such further agents as binders, sweeteners, thickeners, flavouring agents disintegrating agents, coating agents, preservatives, lubricants and/or time delay agents. Suitable sweeteners include sucrose, lactose, glucose, aspartame or saccharine. Suitable disintegrating agents include cornstarch, methylcellulose, polyvinylpyrrolidone, xanthan gum, bentonite, alginic acid or agar. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Certain embodiments of the invention will now be described with reference to the following examples which are intended for the purpose of illustration only.

### EXAMPLES

### Synthetic Protocols

### Preparation of 2-Bromo-7-acetoxy-3-(3,4,5-trimethoxybenzoyl)-6-methoxybenzofuran.

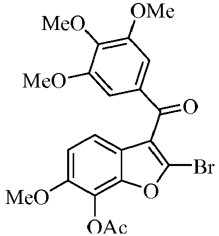

### Step 1: 2-t-Butyldimethylsilyl-3-(t-butyldimethylsilyloxymethylene)-6-methoxy-7-isopropoxybenzofuran (Larock coupling).

A suspension of 2-isopropoxy-3-methoxy-5-iodophenol (4.41 mmol), *1*-(*tert-*butyldimethylsilyl)-3-(*tert*-butyldimethylsilyloxy)propyne (1.5 g, 5.28 mmol), lithium chloride (189 mg, 4.45 mmol) and sodium carbonate (2.34 g, 22.08 mmol) in dry dimethylformamide (5 mL) at 100 °C was deoxygenated 4 times by evacuation and backfilling with nitrogen. Palladium acetate (135 mg, 0.60 mmol) was added and the reaction vessel was degassed twice with nitrogen. The reaction mixture was then stirred at this temperature for 4 hours (tlc) and the solvent was removed by distillation under vacuum. The residue was dissolved in ethyl acetate (75 mL), stirred well, filtered and treated with triethylamine (5 mL). The solution was concentrated onto silica gel (10 g) and purified by flash chromatography (silica gel, eluent = hexane/diethyl ether/triethylamine; 95:5:1%) to afforded the title compound as a yellow oil (1.45 g, 96 %); ¹H NMR (300 MHz, CDCl₃) δ 7.24(d, 1H, *J* = 8.45 Hz), 6.88(d, 1H, *J* = 8.47 Hz), 4.80(s, 2H, CH₂), 4.73(m, 1H), 3.88(s, 3H, OMe), 1.36(d, 6H, *J* = 6.17 Hz), 0.94(s, 9H), 0.92(s, 9H), 0.35(s, 6H), 0.12(s, 6H).

### Step 2: 2-t-Butyldimethylsilyl-3-formyl-6-methoxy-7-isopropoxybenzofuran

To a solution of 2-*t*-butyldimethylsilyl-3-(*t*-butyldimethylsilyloxymethylene)-6-methoxy-7-isopropoxybenzofuran (2.69 mmol) in methanol (100 mL) was added concentrated hydrochloric acid (200 µL) and the reaction was stirred for 30 minutes (monitored by tlc), quenched with triethylamine (2 mL) and the solvent removed by distillation under vacuum. The residue was dissolved in dichloromethane (20 mL), washed with water (10 mL), dried over magnesium sulfate, concentrated under vacuum and co-distilled with toluene (20 mL). The crude product was dissolved in dry dichloromethane (4 mL) and added to a stirred solution of Collin's reagent (chromium trioxide (1.01 g), pyridine (1.65 mL) in dry dichloromethane (30 mL)). The suspension was stirred for 10 minutes, filtered and the residue washed with diethyl ether (20 mL). The filtrate was concentrated onto silica (10 g) and purified by flash chromatography (silica gel, eluent = hexane/diethyl-ether/triethylamine (90:9:1) to afford the title compound as a light yellow oil (503 mg, 48%); ¹H NMR (300 MHz, CDCl₃) δ 10.25(s, 1H, CHO), 7.79(d, 1H, *J* = 8.45 Hz), 6.98(d, 1H, *J* = 8.46 Hz), 4.65(m, 1H), 3.89(s, 3H, OMe), 1.35(d, 6H, *J* = 6.17 Hz), 0.97(s, 9H), 0.45(s, 6H).

### Step 3: 2-t-Butyldimethylsilyl-3-(3,4,5-trimethoxybenzoyl)-6-methoxy-7-isopropoxybenzofuran

To a stirred solution of 3,4,5-trimethoxyiodobenzene (377 mg, 1.27 mmol) in dry tetrahydrofuran (1 mL) at -78 °C under nitrogen was added n-butyllithium (795 µL, 1.59 mmol, 2M solution in cyclohexane) and the reaction mixture was stirred at this temperature for 40 minutes. After this time a solution of 2-*t*-butyldimethylsilyl-3-formyl-6-methoxy-7-isoproxybenzofuran (1.07 mmol) in dry tetrahydrofuran (1 mL) was added to the reaction dropwise via syringe pipette. The reaction mixture was stirred at -60 °C for 20 minutes and then allowed to warm to 0°C, stirred for 10 minutes, quenched with saturated ammonium chloride solution (2 mL) and diluted with ethyl acetate (20 mL). The organic layer was washed with water (10 mL), dried over magnesium sulfate and the solvent was removed under vacuum to give a residue that was co-distilled with toluene. The crude product (908 mg) was dissolved in dry tetrahydrofuran (10 mL) and treated with 2,3-dichloro-5,6-dicyano-1,4-benzoquinone (900 mg, 1.59 mmol) was added. The reaction mixture was stirred at room temperature for 16 hours (monitored by tlc) and then loaded onto silica (10 g) and purified by flash chromatography (silica gel, eluent = hexane/diethyl ether/triethylamine, 90:9:1) to afford the title compound as a light yellow oil (498 mg, 69%); ¹H NMR (300 MHz, CDCl₃) δ 7.14(s, 2H, benzoyl Hs), 6.81(d, 1H, *J* = 8.64 Hz), 6.77(d, 1H, *J* = 8.64 Hz) 4.74(m, 1H), 3.93(s, 3H, OMe), 3.86(s, 3H, OMe), 3.78(s, 6H, 2 x OMe), 1.39(d, 6H, *J* = 6.14 Hz), 1.01(s, 9H), 0.26(s, 6H).

### Step 4: 2-(tert-butyldimethylsilyloxy)-7-acetoxy-3-(3,4,5-trimethoxybenzoyl)-6-methoxybenzofuran

To a stirred solution of 2-(*t*-butyldimethylsilyloxy)-7-isopropoxy-3-(3,4,5-trimethoxybenzoyl)-6-methoxy-benzofuran (160 mg, 0.31 mmol) in dry DCM (2 mL) at room temperature under nitrogen was added solid aluminium trichloride (83 mg, 0.62 mmol) and the reaction mixture was stirred for 15 minutes (monitored by tlc). The reaction was quenched with a saturated solution of ammonium chloride, extracted with dichloromethane and dried over magnesium sulfate. The solvent was removed by distillation and residue was dried by azeotropic removal of water with toluene. The crude product was dissolved in pyridine (2 mL), acetic anhydride (1 mL) was added and reaction mixture was stirred for 2 hours at room temperature. The solvent was distilled under vacuum and the residue was loaded onto silica gel (1 g) and purified by column chromatography (silica gel, eluent, hexane:diethyl-ether; 80:20) (134 mg, 84%); ¹H NMR (300 MHz, CDCl₃) δ 7.14(s, 2H, benzoyl Hs), 6.98(d, 1H, *J* = 8.72 Hz), 6.85(d, 1H, *J* = 8.72 Hz), 3.93(s, 3H, OMe), 3.86(s, 3H, OMe), 3.80(s, 6H, 2 x OMe), 2.41(s, 3H), 0.99(s, 9H), 0.25(s, 6H).

### Step 5: 2-Bromo-7-acetoxy-3-(3,4,5-trimethoxybenzoyl)-6-methoxybenzofuran

To a stirred solution of 2-t-butyldimethylsilyl-7-acetoxy-3-(3,4,5-trimethoxybenzoyl)-6-methoxybenzofuran (120 mg, 0.44 mmol) in 1,2-dichloroethane (1 mL) at room temperature under nitrogen was added bromine (12 µl, 0.44 mmol) dropwise and the reaction mixture was stirred at this temperature for 10 minutes. After this time the reaction was quenched with saturated sodium thiosulfate solution, extracted with ethyl acetate (20 mL), dried over magnesium sulfate and the solvent removed by distillation under vacuum. The crude product was purified by silica gel column chromatography (eluent = Hexane:diethyl ether; 8:2 - 7:3) to afford the title compound as a colourless crystalline solid (91 mg, 81%); ¹H NMR (300 MHz, CDCl₃) δ 7.40(d, 1H, *J* = 8.70 Hz), 7.14(s, 2H, benzoyl-Hs), 6.98(d, 1H, *J=* 8.75 Hz), 3.94(s, 3H, OMe), 3.89(s, 3H, OMe), 3.86(s, 6H, 2 x OMe), 2.43(s, 3H); ¹³C NMR (75 MHz, CDCl₃) δ 187.95(CO), 167.71, 152.75, 149.54, 147.49, 142.59, 131.92, 131.80, 123.91, 121.84, 119.89, 117.72, 109.89, 106.92, 60.69, 56.61, 56.00, 20.09.

### Example 1

### Preparation of 2-Methyl-7-hydroxy-3-(3,4,5-trimethoxybenzoyl)-6-methoxybenzofuran (BNC105)

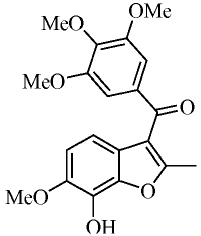

### Preparation A

To a stirred solution of 2-Bromo-7-acetoxy-3-(3,4,5-trimethoxybenzoyl)-6-methoxybenzofuran (20 mg, 0.042 mmol), methyl-boronic acid (40 mg, 0.67 mmol), in 1,4-dioxane (2 mL) at 90 °C was added *tetrakis*-triphenylphosphine palladium (11 mg, 0.01 mmol) followed by the addition of a solution of sodium bicarbonate (40 mg, 0.48 mmol) in distilled water (0.5 mL). The reaction mixture turned red after 5 minutes. After 2 hours (tlc) the reaction mixture was brought to room temperature and was added saturated ammonium chloride (2 mL) and diluted with dichloromethane (20 mL). The organic layer was separated and washed with water, dried over magnesium sulfate and the solvent was removed by distillation under vacuum. The residue was purified by PTLC (eluent = Dichloromethane/Methanol, 1:1) to give the title compound (acetate cleaved during reaction) as a fluffy white solid; (3 mg, 19%).

### Preparation B (Negishi Coupling)

To a stirred solution of zinc-bromide (592 mg, 2.63 mmol) in dry THF(1.5 mL) at 0°C was added the solution of methyl lithium (1.6 M solution in diethyl-ether, 2.6 mL, 4.15 mmol) and the reaction mixture was stirred for 2 hours. Solid 2-bromo-7-acetoxy-3-(3,4,5-trimethoxybenzoyl)-6-methoxy-benzofuran (300 mg, 0.63 mmol) was added and the ether was removed under vacuum and to the rest suspension was added dichlorobis(triphenylphosphine)palladium catalyst (21 mg) and catalytic amount of copper (I) iodide. The reaction mixture was stirred at room temperature for 36 hours (monitored by tlc), quenched with saturated ammonium chloride solution and extracted with dichloromethane (10 mL), dried over magnesium sulfate and solvent distilled under vacuum and the product was purified by silica gel column (eluent = hexane/ethyl acetate; 8:2). The product was crystallized in methanol (106 mg, 46%); ¹H NMR (300 MHz, CDCl₃) δ 7.09(s, 2H, benzoyl Hs), 6.93(d, 1H, *J* = 8.54 Hz), 6.83(d, 1H, *J* = 8.56 Hz),

5.70(bs, 1H, OH), 3.93(s, 3H, OMe), 3.92(s, 3H, OMe), 3.83(s, 6H, 2 x OMe), 2.54(s, 3H, 2-Me)

### Example 2

### Preparation of Disodium 6-methoxy-2-methyl-3-(3,4,5-trimethoxybenzoyl)benzofuran-7-yl phosphate

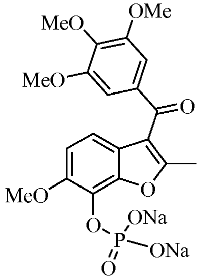

### Step 1: Dibenzyl 6-methoxy-2-methyl-3-(3,4,5-trimethoxybenzoyl)benzofuran-7-yl phosphate:

To a mixture of 0.081 g (0.22 mmol) of (7-hydroxy-6-methoxy-2-methylbenzofuran-3-yl)(3,4,5-trimethoxyphenyl)methanone, 0.086 g (0.261 mmol) of carbon tetrabromide and 0.063 ml (0.283 mmol) of dibenzylphosphite in 2.5 ml of anhydrous acetonitrile 0.046 ml of anhydrous triethylamine was added dropwise at 0°C under nitrogen atmosphere. The resulting mixture was stirred for 2h at room temperature, then diluted to 20 ml with ethyl acetate, washed with water brine, dried over anhydrous magnesium sulfate, filtered off and evaporated to dryness under reduced pressure. The residue was purified by flash column chromatography (dichloromethane/ ethyl acetate, 9:1) to give the title compound as a colorless foam (0.13g, 94%); ¹H NMR (CDCl₃) δ 2.42 (s, 3H, Me-2); 3.83 (s, 1H, OMe); 3.93 (s, 3H, OMe); 5.33 (m, 4H, CH₂Ph); 6.89 (d, CH aromatic, *J* = 8.7 Hz); 7.21 (dd, 1H, CH aromatic, *J* = 8.72 Hz; *J* = 1.2 Hz); 7.08 (s, 2H, CH aromatic); 7.29 - 7.43 (m, 10 H, CH aromatic).

### Step 2: Disodium 6-methoxy-2-methyl-3-(3,4,5-trimethoxybenzoyl)benzofuran-7-yl phosphate:

To a stirred solution of 0.122 g (0.193 mmol) of the product from Step 1 in 1 ml of anhydrous acetonitrile 0.075 ml (0.58 mmol) of bromotrimethylsilane was added at - 5°C under nitrogen atmosphere. The resulting mixture was stirred for 1 h at 0°C, then evaporated to dryness *in vacuo.* The residue was diluted to 5 ml with anhydrous methanol and pH of the solution was brought up about 10 by the addition of sodium methoxide. After evaporation of the resulting mixture under reduced pressure the solid residue was washed with anhydrous isopropanol (4 x 1.5 ml) and anhydrous ethanol (3 x 1.5 ml) and dried under vacuum to give 0.062 g (65 % yield) of title compound as an colorless solid; ¹H NMR (D₂O) δ 2.37 (s, 3H, Me-2); 3.76 (s, 6H, OMe); 3.79 (s, 3H, OMe); 3.82 (s, 3H, OMe); 4.66 (s, H₂O); 6,93 (d, 1H, CH aromatic, *J* = 8.6 Hz); 7.04 (d, 1H, CH aromatic, *J* = 8.6 Hz); 7.10 (s, 2H, CH aromatic).

### Biological data

BNC105P was shown to increase tumour hypoxia by disruption of tumour vasculature. This effect was demonstrated in a wide range of tumours including MDA-MB-231 breast carcinoma, Calu-6 lung carcinoma, Colo205 colon carcinoma and DU-145 prostate carcinoma. With MDA-MB-231 tumours BNC105P was shown to cause release of the angiogenic growth factor VEGF, destruction of tumour endothelial cells, damage of blood vessel integrity and an increase in apoptotic cells.

BNC105 induced vascular shutdown in RENCA renal tumours with an increase GLUT1, HIF-1a, PERK, eIF2a and VEGF. In vascular shutdown in RENCA renal tumours induced by BNC105 BNC105 induced hypoxia with an increase in CXCL12 and MMP-9 with an inflammatory immuno-response and induced chemotaxis.

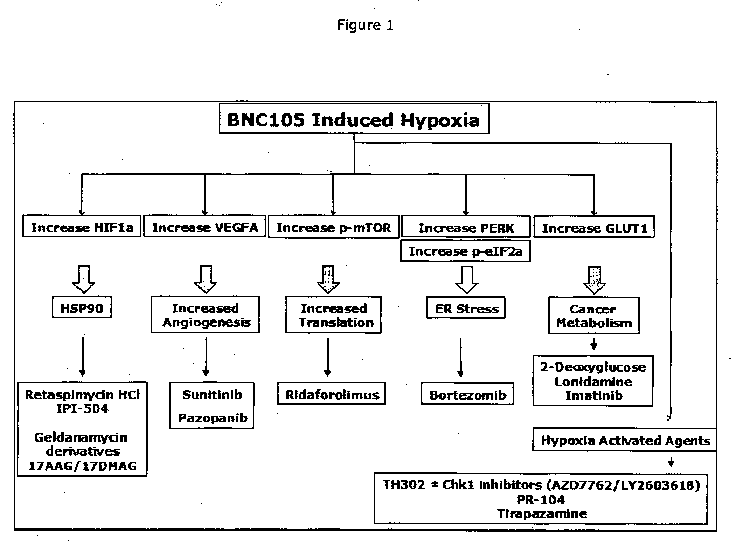

These findings lead to the present inventors to examine combination therapies involving BNC105 and a range of tumour hypoxia targeting agents. The rationale behind this approach is summarised in Figure 1.

### Combination therapy of BNC105 with Pazopanib

Based on investigations on the histological changes caused by BNC105 antitumor action, it was established that >95% of the tumor mass becomes necrotic, while peripheral regions remain viable. Increased expression of a number of proteins that drive tumor recovery were observed in the viable regions. Among these is the angiogenesis growth factor VEGF. The present inventors sought to investigate the potential therapeutic benefit of combining BNC105 with an inhibitor of the VEGF/VEGFR2 signalling pathway.

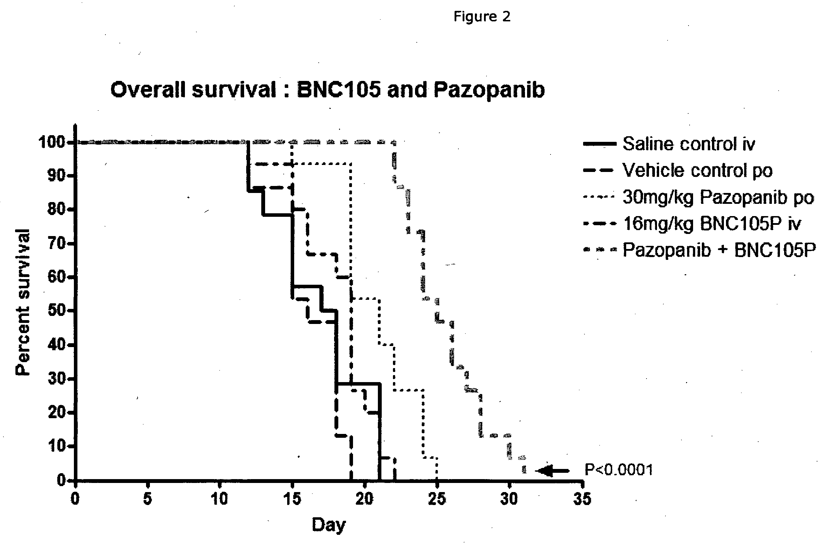

Pazopanib is a Tyrosine Kinase Inhibitor that suppresses signalling through the VEGF family receptors and has been approved by the FDA for first line therapy use in renal cancer. Following establishment of a safe treatment regimen, the present inventors examined the anti-cancer effects of co-administering BNC105 and Pazopanib in the RENCA model involving mice carrying kidney tumors. Mice were treated with one cycle of BNC105 (16 mg/kg/dose on Day 2 and Day 9 in a 21 day cycle) while receiving daily oral administrations of Pazopanib (Days 1-21; 30 mg/kg). Treatment was discontinued at the 21-Day mark and the animals monitored for overall survival. Based on the data obtained combining BNC105 treatment with Pazopanib resulted in a considerable and statistically significant increase in animal survival (Figure 2).

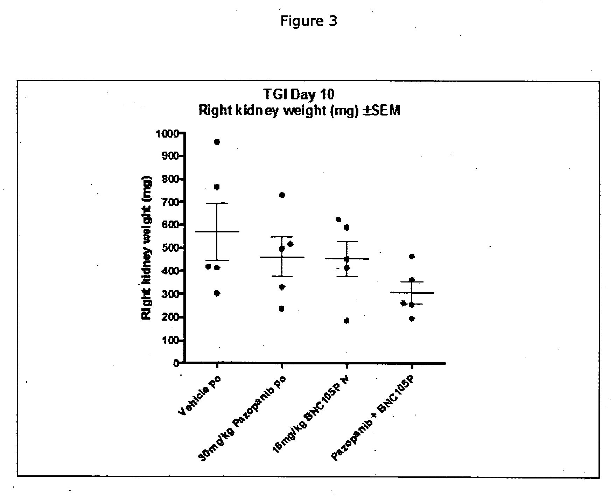

RENCA (VHL wildtype) orthotopic tumour bearing kidneys from Balb/c mice were weighed on Day 10 day of treatment (Pazopanib 30mg/kg, p.o. Daily, BNC105 16mg/kg i.v. Days 2 and 9). Concurrent treatment with BNC105 and Pazopanib resulted in 47% tumour growth inhibition compared to 21 or 19% inhibition with BNC105 or Pazopanib treatment alone respectively. These results are shown in Figure 3.

As is demonstrated by these results treatment of RENCA orthotopic tumours with BNC105 + Pazopanib resulted in tumour growth inhibition and an overall survival greater than in animals treated with Pazopanib or BNC105 alone. This shows that the combination therapy of BNC105 and Pazopanib is much more effective than BNC105 or Pazopanib monotherapy.

### Combination therapy of BNC105 with Bortezomib:

Formalin fixed paraffin embedded RENCA (VHL wildtype) orthotopic tumour sections from tumour bearing Balb/c mice collected 24 hours after a single dose i.v. 32mg/kg BNC105P showed upregulation of unfolded protein response markers PERK (Cell signalling #5683) and phosphorylation of eIF2α (Cell Signalling #5324). (Fast Red detection on hematoxylin)

RENCA orthotopic tumour bearing kidneys from Balb/c mice were photographed 4 hours after dosing BNC105 on Day 9 of treatment (Bortezomib 0.5mg/kg iv Days 1, 5, 8, BNC105 32mg/kg iv Days 2 and 9). Formalin fixed and paraffin embedded sections were stained using a TUNEL assay (Roche) to visualize cell necrosis. Very little tumor necrosis was seen with each of the drugs used as monotherapies. Clearly increased necrosis, visualised as increased TUNEL staining, was seen in tumors extracted from mice treated with the combination of Bortezomib+BNC105.

Treatment of RENCA tumours with BNC105 + Bortezomib resulted in increased tumour necrosis compared to animals treated with Bortezomib or BNC105 alone. This demonstrates a clear advantage in the use of the BNC105 Bortezomib combination therapy over BNC105 or Bortezomib monotherapy.

## Claims

1. A pharmaceutical combination for use in treating a proliferative disease selected from renal cancer, lung cancer, breast cancer, prostate cancer, and pancreatic cancer, comprising:
a) a vascular disrupting agent (VDA) which is a compound having the structure 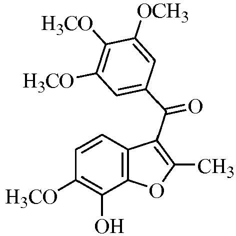 or a salt or solvate thereof, or 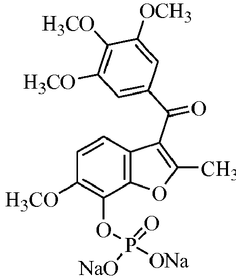 or a salt or solvate thereof; and
(b) at least one hypoxia targeting agent selected from Pazopanib, Bortezomib or TH-302.

2. A pharmaceutical combination for use according to claim 1 wherein the VDA is a compound having the structure 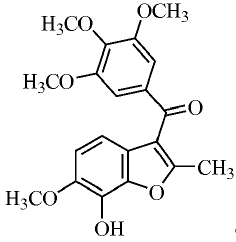 or a salt or solvate thereof.

3. A pharmaceutical combination for use according to claim 1 wherein the VDA is a compound having the structure 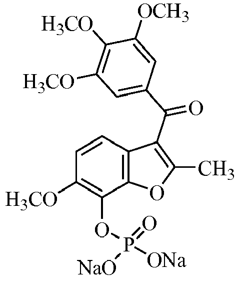 or a salt or solvate thereof

4. A pharmaceutical combination for use according to claim 1, 2 or 3 wherein the proliferative disease is renal cancer.

5. A kit of parts comprising a pharmaceutical combination for use according to any one of claims 1 to 4.

## Patentansprüche

1. Pharmazeutische Kombination zur Verwendung beim Behandeln einer proliferativen Krankheit, ausgewählt aus Nierenkrebs, Lungenkrebs, Brustkrebs, Prostatakrebs und Pankreaskrebs, umfassend:
a) ein vaskuläres Disruptionsmittel (VDA), das eine Verbindung ist, die die folgende Struktur hat 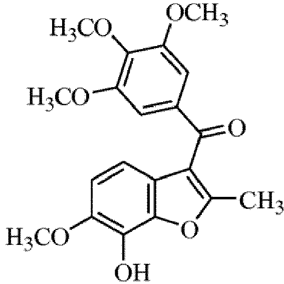 oder ein Salz oder Solvat davon, oder 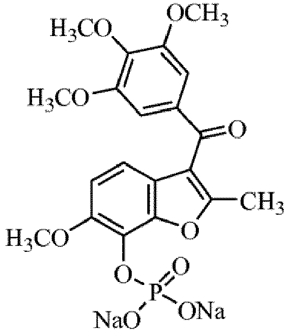 oder ein Salz oder Solvat davon; und
(b) mindestens ein Hypoxie-Targetingmittel, ausgewählt aus Pazopanib, Bortezomib oder TH-302.

2. Pharmazeutische Kombination zur Verwendung nach Anspruch 1, wobei das VDA eine Verbindung ist, die die folgende Struktur hat 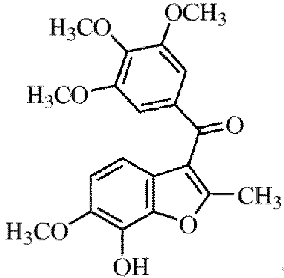 oder ein Salz oder Solvat davon.

3. Pharmazeutische Kombination zur Verwendung nach Anspruch 1, wobei das VDA eine Verbindung ist, die die folgende Struktur hat 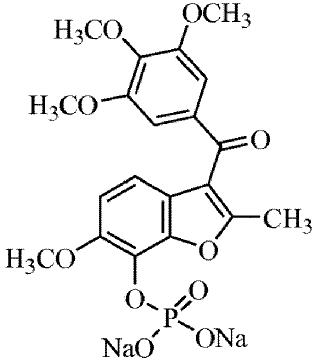 oder ein Salz oder Solvat davon.

4. Pharmazeutische Kombination zur Verwendung nach Anspruch 1, 2 oder 3, wobei die proliferative Krankheit Nierenkrebs ist.

5. Satz von Teilen, umfassend eine pharmazeutische Kombination zur Verwendung nach einem der Ansprüche 1 bis 4.

## Revendications

1. Combinaison pharmaceutique pour une utilisation dans le traitement d'une maladie proliférative choisie parmi le cancer du rein, le cancer du poumon, le cancer du sein, le cancer de la prostate et le cancer du pancréas, comprenant :
(a) un agent vasculotoxique (VDA) qui est un composé ayant la structure 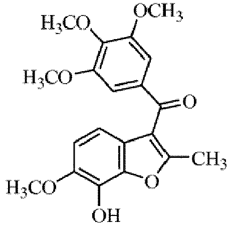 ou un sel ou solvate de celui-ci, ou 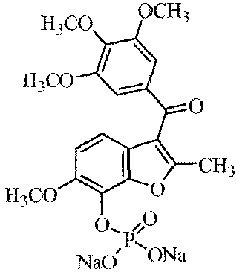 ou un sel ou solvate de celui-ci ; et
(b) au moins un agent ciblant l'hypoxie choisi parmi le pazopanib, le bortézomib ou TH-302.

2. Combinaison pharmaceutique pour une utilisation selon la revendication 1, dans laquelle le VDA est composé ayant la structure 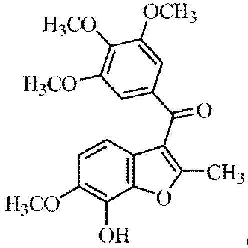 ou un sel ou solvate de celui-ci.

3. Combinaison pharmaceutique pour une utilisation selon la revendication 1, dans laquelle le VDA est composé ayant la structure 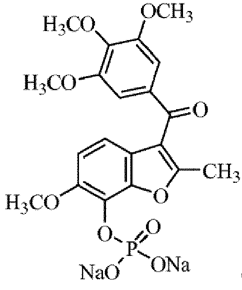 ou un sel ou solvate de celui-ci.

4. Combinaison pharmaceutique pour une utilisation selon la revendication 1, 2 ou3, dans laquelle la maladie proliférative est le cancer du rein.

5. Kit de pièces comprenant une combinaison pharmaceutique pour une utilisation selon l'une quelconque des revendications 1 à 4.
